# EUROPEAN PATENT SPECIFICATION

(11) **EP 2 663 343 B1**
(45) Date of publication and mention of the grant of the patent: **06.12.2017**
(21) Application number: 12734153.5
(22) Date of filing: 09.01.2012
(51) Int. Cl.: A61L 27/56, A61F 2/01, A61F 2/02, A61L 27/40, A61F 2/44, A61L 27/06, A61L 27/50, A61F 2/30, A61F 2/36, A61B 17/80

(54) **DEVICES AND METHODS FOR TISSUE ENGINEERING**
VORRICHTUNGEN UND VERFAHREN ZUR GEWEBEZÜCHTUNG
DISPOSITIFS ET PROCÉDÉS D'INGÉNIERIE TISSULAIRE

(30) Priority: 12.01.2011 US 201161431996 P
(43) Date of publication of application: 20.11.2013
(73) Proprietor: Bio2 Technologies, Inc., Woburn, MA 01801 (US)
(72) Inventor: LIU, James, Jenq, Mason, OH 45040 (US); KREVOLIN, Janet, L., Somerville, MA 02143 (US)
(74) Representative: Viering, Jentschura & Partner mbB Patent- und Rechtsanwälte
(86) International application number: PCT/US2012/020578
(87) International publication number: WO 2012/096858

(56) References cited:
- WO-A2-2006/106506
- US-A1- 2005 177 238
- US-A1- 2006 216 321
- US-A1- 2007 111 878
- US-A1- 2007 150 068
- US-A1- 2009 035 511
- US-A1- 2009 157 182
- US-A1- 2009 289 387
- US-A1- 2009 289 387
- US-A1- 2010 042 226
- US-B1- 6 187 329

## Description

### Field of the Invention

The present invention relates generally to the field of porous medical implants and prosthetic devices. More specifically, the invention relates to a tissue engineering scaffold having a gradient of properties.

### Background of the Invention

Prosthetic devices are often required for repairing defects in bone tissue in surgical and orthopedic procedures. Prostheses are increasingly required for the replacement or repair of diseased or deteriorated bone tissue in an aging population and to enhance the body's own mechanism to produce rapid healing of musculoskeletal injuries resulting from severe trauma or degenerative disease.

Synthetic prosthetic devices are used to partially or completely replace bone joints or bone segments in the repair of defects. Knowledge of the structure and bio-mechanical properties of bone and an understanding of the natural bone healing process provides guidance on the desired properties and characteristics of an ideal synthetic prosthetic device for such bone repair. These characteristics include, without limitation, osteostimulation and/or osteoconductivity to promote the ingrowth of natural bone tissue into the device as the repair heals; and load bearing or weight sharing to support the repair site and to exercise the tissue surrounding the repair to promote the growth of durable and healthy bone tissue.

Materials for synthetic prosthetic devices and tissue engineering scaffolds developed to date have been successful in attaining at least a portion of the desired characteristics of an ideal tissue scaffold but nearly all materials compromise at least some aspect of the bio-mechanical requirements that leads to increased probability of fixation issues and/or an increased probability of premature failure of the implanted device.

WO 2006/106506 A2 discloses a medical scaffold comprising an electrospun element consisting of PCL and PLA polymers and/or copolymers.

This document discloses discloses a tissue engineering scaffold comprising: a first region of interconnected and bonded fiber, the first region having a porosity exhibiting a first value; a second region of interconnected and bonded fiber, the second region having the porosity exhibiting a second value; a third region of interconnected and bonded fiber between the first region and the second region, the third region having the property exhibiting a gradient value between the first value and the second value. US 2007/111878 A1 discloses an extrudable mixture for producing a highly porous substrate, wherein fibers, such as organic, inorganic, glass, ceramic or metal fibers, are mixed into a mass that when extruded and cured, forms a highly porous substrate.

### Brief Summary of the Invention

The present invention meets the objectives of an effective synthetic bone prosthetic for the repair of bone defects by providing a tissue engineering scaffold that has a first region of interconnected and bonded fiber with a property exhibiting a first value, and a second region of interconnected and bonded fiber with the property exhibiting a second value. An interfacial third region is formed between the first region and the second region with the property exhibiting a gradient value between the first value and the second value. Embodiments of the invention include, without limitation, tissue engineering scaffolds where the property is porosity, elastic modulus, strength, composition and bioactivity. The claimed scaffold is sized for engagement within a space between adjacent vertebrae and to maintain the intervertebral space.

Other exemplary embodiments are described that include, without limitation, an acetabular cup, and an osteotomy wedge implant.

Methods of fabricating a tissue engineering scaffold with a desired property having a value spatially distributed in at least two regions is provided. The methods include providing two batch materials, a first batch material and a second batch material, each batch material including fiber, binder, and a pore former with the materials having each a respective set of characteristics. The scaffold is formed using the first batch material and the second batch material, with the first batch material associated with a first region and the second batch material associated with a second region, the first region being adjacent to the second region. The formed scaffold is cured to first remove the binder and pore former, and then heated to form bonds between the fiber of the first batch material and the fiber of the second batch material. In this process, a desired property having a first value corresponds to the first region and a second value corresponding to the second region. Specific embodiments of forming the scaffold include, without limitation, co-extrusion, compression rolling, compression molding, injection molding, rapid prototype forming and hot isostatic pressing.

### Brief Description of the Several Views of the Drawings

The foregoing and other objects, features, and advantages of the invention will be apparent from the following detailed description of the several embodiments of the invention as illustrated in the accompanying drawings in which like reference characters refer to the same parts throughout the different views. The drawings are not necessarily to scale, with emphasis instead being placed upon illustrating the principles of the invention.
FIG. 1 shows an optical micrograph at approximately 100X magnification showing an embodiment of a tissue scaffold according to the present invention.
FIG. 2 is a flowchart of an embodiment of a method of the present invention for forming the tissue scaffold of FIG. 1.
FIG. 3 is a flowchart of an embodiment of a method of a preparation step of the method of FIG. 2 invention.
FIG. 4 is a flowchart of an embodiment of a curing step according to the method of FIG. 2 invention.
FIG. 5 is a schematic representation of an embodiment of an object fabricated according to a method of the present invention.
FIG. 6 is a schematic representation of the object of FIG. 5 upon completion of a volatile component removal step of the method of the present invention.
FIG. 7 is a schematic representation of the object of FIG 6 upon completion of a bond formation step of the method of the present invention.
FIG. 8 is a graphic representation of the evaluation of the stress-strain relationship of two components of the materials of the present invention.
FIG. 9 is a schematic representation of an embodiment of a method of the forming step using compression formation of materials according to the present invention.
FIG. 10 is a schematic representation of a tissue scaffold formed as an acetabular cup according to the embodiment of FIG. 9.
FIG. 11 is a graphic representation of the evaluation of a gradient in the properties of a tissue scaffold according to the embodiment of FIG. 9.
FIG. 12 is a schematic representation of an embodiment of a method of the forming step using co-extrusion according to the present invention.
FIG. 13 is a schematic representation of an embodiment of a method of the forming step using rapid prototype dispensing according to the present invention.
FIG. 14 is a schematic representation of a tissue scaffold according to the present invention fabricated into an osteotomy wedge.
FIG. 15 is a schematic representation of a tissue scaffold according to the present invention fabricated into a hip stem.
FIG. 16 is a schematic cross-section of a tissue scaffold according to the present invention having a gradient in porosity for limiting the in-growth of tissue into the scaffold.

While the above-identified drawings set forth presently disclosed embodiments, other embodiments are also contemplated, as noted in the discussion. This disclosure presents illustrative embodiments by way of representation and not limitation. Numerous other modifications and embodiments can be devised by those skilled in the art which fall within the scope and spirit of the principles of the presently disclosed embodiments.

### Detailed Description of the Invention

The present invention provides a synthetic prosthetic tissue scaffold for the repair of tissue defects according to claim 1. As used herein, the terms "synthetic prosthetic tissue scaffold" and "bone tissue scaffold" and "tissue scaffold" and "synthetic bone prosthetic" in various forms may be used interchangeably throughout.

Various types of implants have been developed for tissue engineering applications in an attempt to provide a synthetic prosthetic tissue scaffold device. These devices are designed to meet or exceed the required mechanical properties of the bone segment or joint being replaced while not exceeding the physiological space limitations of the repair. While mechanical designs and material compositions can be specified to meet the desired mechanical properties of the bone or joint, the success and performance of the implant can only be assessed once it has been incorporated into the living tissue.

A challenge for most synthetic implants is the attachment of the implant to the adjacent bone tissue. A synthetic bone prosthetic can be designed with the strength and size required for the indication, but if the synthetic bone prosthetic does not sufficiently attach to the surrounding bone tissue the device may loosen and cause pain, discomfort and require revision. There are four primary methods for fixation of a synthetic prosthetic tissue scaffold. These methods include: mechanical fixation using pins and/or screws; impaction of the device into the medullary cavity of the bone or similar press-fit insertions; cement between the bone and the device using materials such as polymethylmethacrylate materials containing hydroxyapatite; and osteostimulative porous materials that promote in-growth of bone tissue (or a combination thereof). While each method has problems that can lead to fixation issues, the use of osteostimulative porous materials as the interface between bone tissue and the implant provides the highest probability for integration with the surrounding tissue.

Implants into living tissue evoke a biological response dependent upon a number of factors. The composition of the implant has a direct impact on the biological response. Biologically inert materials are commonly encapsulated with fibrous tissue to isolate the implant from the host. Metals and most polymers produce this interfacial response as do nearly inert ceramics such as alumina or zirconia. Biologically active materials or bioactive materials, elicit an additional biological response that can produce an interfacial bond securing the implant material to the living tissue, much like the interface that is formed when natural tissue repairs itself. This interfacial bonding can lead to an interface that stabilizes the implant in the bony bed and provides stress transfer from the implant across the bonded interface into the bone tissue. Once the implant is secured to the surrounding bone material and loads are applied to the repair site, the bone tissue including the regenerated bone tissue that encapsulates or bonds to the implant is stressed, thus limiting bone tissue resorption due to stress shielding. Implants that are surface active or osteoconductive further elicit a biological response that can induce tissue in-growth that can be further enhanced by the pore morphology of the material. An open pore material into which bone can grow is advantageous for integration of the implant into the surrounding tissue.

A bone in its natural state carries its external loads by itself. When subjected to replacement, enhancement or augmentation by a prosthetic implant or device, the load is then carried by the combination of the bone and the implant. Any reduction in stress relative to the natural state of the bone can be a stimulus for the bone to adapt itself by reducing its mass in accordance with the phenomenon known as Wolff's Law. Load bearing implants that are attached to surrounding tissue can minimize the effect of remodeling with mechanical properties including elastic modulus or flexibility that match the surrounding tissue.

Accordingly, a synthetic implant may require regions exhibiting osteoconductive properties, regions of high strength, and regions of low elastic modulus that can not be attained by currently available material or composites. The present invention is directed toward the use of fiber-based structures for a tissue engineering scaffold that can be fabricated with controlled properties associated with specific regions of the device. The term "fiber" as used herein is meant to describe a wire, filament, rod or whisker in a continuous or discontinuous form having an aspect ratio greater than one, and formed from a wire-drawing or fiber-forming process such as drawn, spun, blown, or other similar process typically used in the formation of fibrous materials. Fiber-based structures are generally known to provide high strength-to-weight ratios given that the strength of an individual fiber can be significantly greater than a solid structure of the same composition. A fiber can be produced with relatively few discontinuities that contribute to the formation of stress concentrations for failure propagation. Furthermore, a fiber-based structure provides for stress relief and thus, greater strength, when the fiber-based structure is subjected to strain in that the failure of any one individual fiber does not propagate through adjacent fibers. Accordingly, a fiber-based structure exhibits superior mechanical strength properties over an equivalent size and porosity than a solid material in a porous form of the same composition.

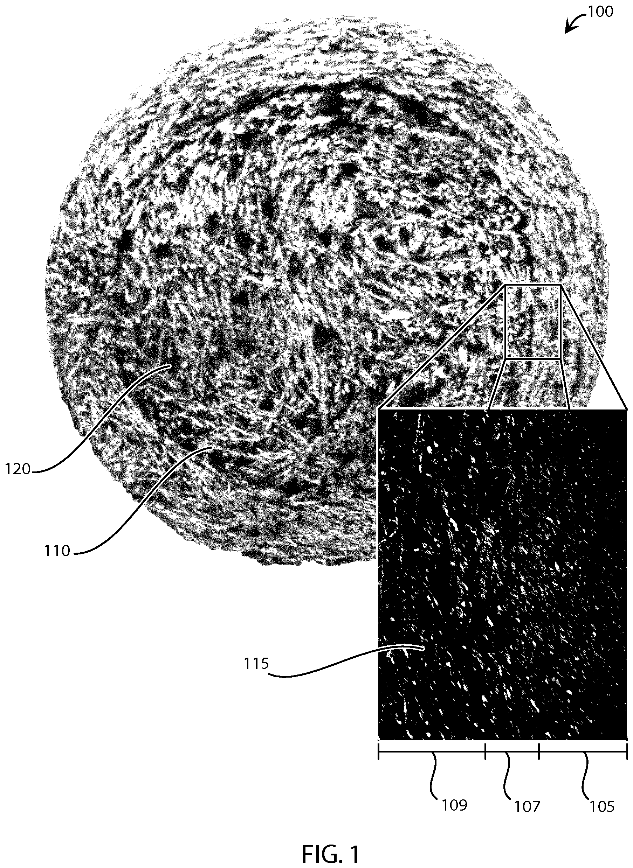

FIG. 1 is an optical micrograph at approximately 50X magnification showing a portion of an embodiment of a tissue scaffold 100 of the present invention. The tissue scaffold 100 is a three-dimensional matrix 110 forming a structure that mimics the mechanical properties of bone structure including strength, elastic modulus, and pore morphology. The scaffold 100 can be a porous material having a network of pores 120 that are generally interconnected. In an embodiment, the interconnected network of pores 120 provide osteconductivity. As used herein, the term osteoconductive means that the material can facilitate the in-growth of tissue. Cancellous bone of a typical human has a compressive crush strength ranging between about 4 to about 12 MPa with an elastic modulus raging between about 0.1 to about 1.0 GPa. As will be shown herein below the tissue scaffold 100 of the present invention can provide a porous tissue scaffold exhibiting a gradient value between two regions with a first region having a property exhibiting a first value and a second region exhibiting a second value, with a third region having the property exhibiting a gradient range of values between the first value and the second value. As shown in FIG. 1, a first region 105 exhibits low porosity and a second region 109 exhibits a high porosity, with a third region 107 exhibiting a transition in porosity from the high porosity of the first region 105 and the second region 109.

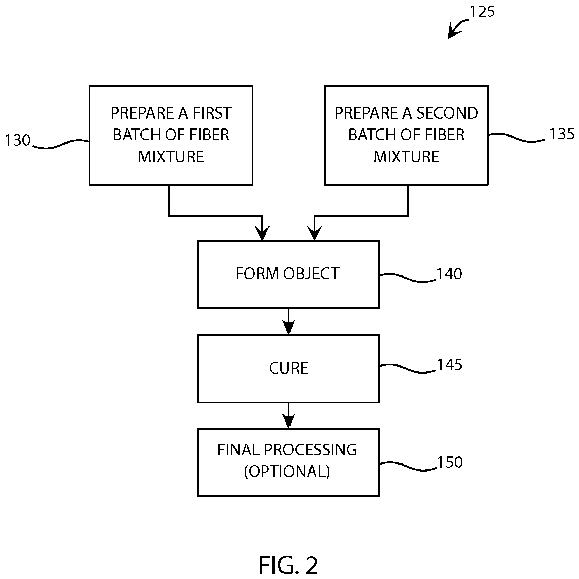

Referring to FIG. 2, a method 100 is shown to provide a tissue engineering scaffold that provides a property exhibiting a gradient value between two regions to provide a first region having a property exhibiting a first value and a second region exhibiting a second value, with a third region having the property exhibiting a gradient range of values between the first value and the second value. In an embodiment of the invention the method 125 starts with the preparation of a first batch of fiber mixture 130 and the preparation of a second batch of fiber mixture 135. The first batch 130 and the second batch 135 are used to form the object 140 and the formed object is cured 145. Optionally, the cured object can receive final processing 150 including machining operations to meet final size dimensional requirements and tolerances.

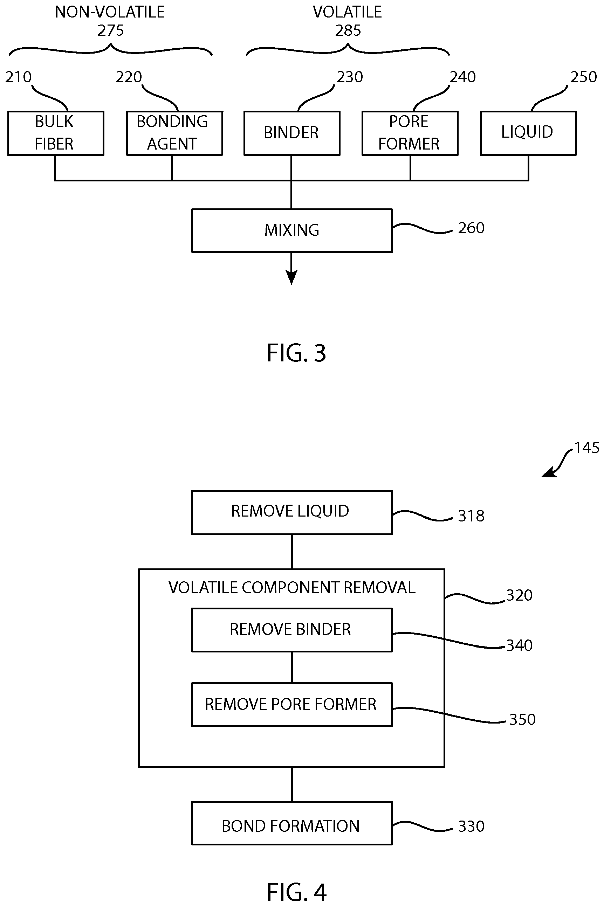

Each of the first batch 130 and the second batch 135 are mixtures of fiber and additives with a liquid that have a plastically deformable consistency to facilitate the forming step 140. Referring to FIG. 3, the preparation steps 130 and 135 of an embodiment of the invention are further described. Generally, bulk fibers 210 are mixed with a binder 230 and a liquid 250 to form a plastically moldable material. The bulk fiber 210 can be provided in bulk form, or as chopped fibers. The diameter of the fiber 210 can range from about 2 to about 500 µm and typically between about 25 to about 200 µm. Fibers 210 of this type are typically produced with relatively narrow and controlled distribution of fiber diameter. Fibers 210 may have a specific diameter or a mixture of fibers having a range or distribution of fiber diameters may be used. The diameter of the fibers 210 will influence the resulting pore size and pore size distribution of the resulting porous structure as well as the size and thickness of the three-dimensional matrix 110, which will influence the osteoconductivity of the scaffold 100 as well as the resulting strength characteristics, including compressive strength and elastic modulus. Additionally, if the composition of the fiber 210 in the first batch 130 or the second batch 135 is a bioactive material, the fiber diameter will influence the rate at which the scaffold 100 would be dissolved by body fluids when implanted in living tissue.

The binder 230 and the liquid 250, when mixed with the fiber 210, create a plastically formable batch mixture that enables the fibers 210 to be evenly distributed throughout the batch while providing green strength to permit the batch material to be formed into the desired shape in the subsequent forming step 140. An organic binder material can be used as the binder 230, such as methylcellulose, hydroxypropyl methylcellulose (HPMC), ethylcellulose and combinations thereof. The binder 230 can include materials such as polyethylene, polypropylene, polybutene, polystyrene, polyvinyl acetate, polyester, isotactic polypropylene, atactic polypropylene, polysulphone, polyacetal polymers, polymethyl methacrylate, fumaron-indane copolymer, ethylene vinyl acetate copolymer, styrenebutadiene copolymer, acryl rubber, polyvinyl butyral, inomer resin, epoxy resin, nylon, phenol formaldehyde, phenol furfural, paraffin wax, wax emulsions, microcrystalline wax, celluloses, dextrines, chlorinated hydrocarbons, refined alginates, starches, gelatins, lignins, rubbers, acrylics, bitumens, casein, gums, albumins, proteins, glycols, hydroxyethyl cellulose, sodium carboxymethyl cellulose, polyvinyl alcohol, polyvinyl pyrrolidone, polyethylene oxide, polyacrylamides, polyethyterimine, agar, agarose, molasses, dextrines, starch, lignosulfonates, lignin liquor, sodium alginate, gum arabic, xanthan gum, gum tragacanth, gum karaya, locust bean gum, irish moss, scleroglucan, acrylics, and cationic galactomanan, or combinations thereof. Although several binders 230 are listed above, it will be appreciated that other binders may be used. The binder 230 provides the desired rheology of the plastic batch material in order to form a desired object and maintaining the relative position of the fibers 210 in the mixture while the object is formed, while remaining inert with respect to the fiber materials. The physical properties of the binder 230 will influence the pore size and pore size distribution of the pore space 120 of the scaffold 100. Preferably, the binder 230 is capable of thermal disintegration, or selective dissolution, without impacting the chemical composition of the bioactive components, including the fiber 210.

The liquid 250 is added as needed to attain a desired rheology in the plastic batch material suitable for forming the plastic batch material into the desired object in the subsequent forming step 140. Water is typically used, though solvents of various types can be utilized. Rheological measurements can be made during the mixing step 260 to evaluate the plasticity and cohesive strength of the mixture prior to the forming step 140.

Pore formers 240 can be included in the mixture to enhance the pore space 120 of the scaffold 100. Pore formers are non-reactive materials that occupy volume in the plastic batch material during the mixing step 260 and the forming step 270. When used, the particle size and size distribution of the pore former 240 will influence the resulting space between fibers impacting the pore size and pore size distribution of the pore space 120 of the scaffold 100. Particle sizes can typically range between about 25 µm or less to about 450 µm or more, or alternatively, the particle size for the pore former can be a function of the fibers 210 diameter ranging from about 0.1 to about 100 times the diameter of the fibers 210. The pore former 240 must be readily removable during the curing step 145 without significantly disrupting the relative position of the surrounding fibers 210. In an embodiment of the invention, the pore former 240 can be removed via pyrolysis or thermal degradation, or volatilization at elevated temperatures during the curing step 280. For example, microwax emulsions, phenolic resin particles, flour, starch, or carbon particles can be included in the mixture as the pore former 240. Other pore formers 240 can include carbon black, activated carbon, graphite flakes, synthetic graphite, wood flour, modified starch, celluloses, coconut shell husks, latex spheres, bird seeds, saw dust, pyrolyzable polymers, poly (alkyl methacrylate), polymethyl methacrylate, polyethyl methacrylate, poly n-butyl methacrylate, polyethers, poly tetrahydrofuran, poly (1,3-dioxolane), poly (alkalene oxides), polyethylene oxide, polypropylene oxide, methacrylate copolymers, polyisobutylene, polytrimethylene carbonate, poly ethylene oxalate, poly beta-propiolactone, poly delta-valerolactone, polyethylene carbonate, polypropylene carbonate, vinyl toluene/alpha-methylstyrene copolymer, styrene/alpha-methyl styrene copolymers, and olefin-sulfur dioxide copolymers. Pore formers 240 may be generally defined as organic or inorganic, with the organic typically burning off at a lower temperature than the inorganic. Although several pore formers 240 are listed above, it will be appreciated that other pore formers 240 may be used. Pore formers 240 can be, though need not be, fully biocompatible since they are removed from the scaffold 100 during processing.

A bonding agent 220 can be included in the mixture to promote bond formation and the performance of the resulting scaffold 100. The bonding agent 220 can include powder-based material of the same composition as the bulk fiber 210, or it can include powder-based material of a different composition. As will be explained in further detail below, the bonding agent 220 based additives enhance the bonding strength of the intertangled fibers 210 forming the three-dimensional matrix 110 through the formation of bonds between adjacent and intersecting fibers 210. The bonding agent 220 can be bioinert metal, glass, glass-ceramic, ceramic, or precursors thereto. In an embodiment of the present invention, the bonding agent 220 is calcium phosphate. In alternative embodiments, the bonding agent 220 is beta-tricalcium phosphate. In yet another alternative embodiment, the bonding agent 220 is hydroxyapatite. In yet another alternative embodiment, the bonding agent 220 is a bioactive glass or precursors thereto.

The relative quantities of the respective materials, including the bulk fiber 210, the binder 230, and the liquid 250 depend on the overall porosity desired in the tissue scaffold 100. For example, to provide a scaffold 100 having approximately 60% porosity, the nonvolatile components 275, such as the fiber 210, would amount to approximately 40% of the mixture by volume. The relative quantity of volatile components 285, such as the binder 230 and the liquid 250 would amount to approximately 60% of the mixture by volume, with the relative quantity of binder to liquid determined by the desired rheology of the mixture. Furthermore, to produce a scaffold 100 having porosity enhance by the pore former 240, the amount of the volatile components 285 is adjusted to include the volatile pore former 240. Similarly, to produce a scaffold 100 having strength enhanced by the bonding agent 220, the amount of the nonvolatile components 275 would be adjusted to include the nonvolatile bonding agent 220. It can be appreciated that the relative quantities of the nonvolatile components 275 and volatile components 285 and the resulting porosity of the scaffold 100 will vary as the material density may vary due to the reaction of the components during the curing step 280. Specific examples are provided herein below.

In the mixing step 260, the fiber 210, the binder 230, the liquid 250, the pore former 240 and/or the bonding agent 220, if included, are mixed into a homogeneous mass of a plastically deformable and uniform mixture. The mixing step 260 may include dry mixing, wet mixing, shear mixing, and kneading, which can be necessary to evenly distribute the material into a homogeneous mass while imparting the requisite shear forces to break up and distribute or de-agglomerate the fibers 210 with the non-fiber materials. The amount of mixing, shearing, and kneading, and duration of such mixing processes depends on the selection of fibers 210 and non-fiber materials, along with the selection of the type of mixing equipment used during the mixing step 260, in order to obtain a uniform and consistent distribution of the materials within the mixture, with the desired rheological properties for forming the object in the subsequent forming step 270. Mixing can be performed using industrial mixing equipment, such as batch mixers, shear mixers, and/or kneaders.

Referring back to FIG. 2, each of the first batch 130 and the second batch 135 are provided as a plastically deformable homogeneous batch material mixture that are cooperatively formed into an object at step 140. The forming step 140 can include, without limitation, extrusion, rolling, pressure casting or shaping into nearly any desired form using the first batch 130 and the second batch 135, examples of which are provided in further detail herein below. The forming step 140 creates the object with at least two regions of the respective batch materials and creates a third region at the transition between batch materials where the first batch 130 and the second batch 135 combine.

The object is then cured into the tissue scaffold 100 in the curing step 145 as further described with reference to FIG. 4. The curing step 145 can be performed as the sequence of three phases: a drying step 310; a volatile component removal step 320; and a bond formation step 330. In the first phase, drying 310, the formed object is dried by removing the liquid using slightly elevated temperature heat with or without forced convection to gradually remove the liquid. Various methods of heating the object can be used, including, but not limited to, heated air convection heating, vacuum freeze drying, solvent extraction, microwave or electromagnetic/ radio frequency (RF) drying methods. The liquid within the formed object is preferably not removed too rapidly to avoid drying cracks due to shrinkage. Typically, for aqueous based systems, the formed object can be dried when exposed to temperatures between about 90°C and about 150°C for a period of about one hour, though the actual drying time may vary due to the size and shape of the object, with larger, more massive objects taking longer to dry. In the case of microwave or RF energy drying, the liquid itself, and/or other components of the object, adsorb the radiated energy to more evenly generate heat throughout the material. During the drying step 310, depending on the selection of materials used as the volatile components, the binder 230 can congeal or gel to provide greater green strength to provide rigidity and strength in the object for subsequent handling.

Once the object is dried, or substantially free of the liquid component 250 by the drying step 310, the next phase of the curing step 280 proceeds to the volatile component removal step 320. This phase removes the volatile components (e.g., the binder 230 and the pore former 240) from the object leaving only the non-volatile components that form the three-dimensional matrix 110 of the tissue scaffold 100. The volatile components can be removed, for example, through pyrolysis or by thermal degradation, or solvent extraction. The volatile component removal step 320 can be further parsed into a sequence of component removal steps, such as a binder burnout step 340 followed by a pore former removal step 350, when the volatile components 285 are selected such that the volatile component removal step 320 can sequentially remove the components. For example, HPMC used as a binder 230 will thermally decompose at approximately 300°C. A graphite pore former 220 will oxidize into carbon dioxide when heated to approximately 600°C in the presence of oxygen. Similarly, flour or starch, when used as a pore former 220, will thermally decompose at temperatures between about 300°C and about 600°C. Accordingly, the formed object composed of a binder 230 of HPMC and a pore former 220 of graphite particles, can be processed in the volatile component removal step 320 by subjecting the object to a two-step firing schedule to remove the binder 230 and then the pore former 220. In this example, the binder burnout step 340 can be performed at a temperature of at least about 300°C but less than about 600°C for a period of time. The pore former removal step 350 can then be performed by increasing the temperature to at least about 600°C with the inclusion of oxygen into the heating chamber. This thermally-sequenced volatile component removal step 320 provides for a controlled removal of the volatile components 285 while maintaining the relative position of the non-volatile components 275 in the formed object.

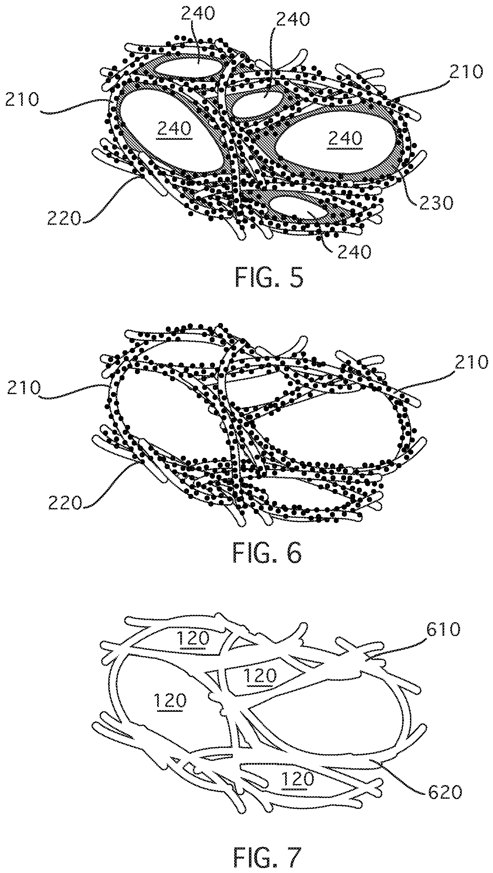

FIG. 5 depicts a schematic representation of the various components of the formed object prior to the volatile component removal step 320. The fibers 210 are intertangled within a mixture of the binder 230 and the pore former 240. Optionally, the bonding agent 220 can be further distributed in the mixture. FIG. 6 depicts a schematic representation of the formed object upon completion of the volatile component removal step 320. The fibers 210 maintain their relative position as determined from the mixture of the fibers 210 with the volatile components 285 as the volatile components 285 are removed. Upon completion of the removal of the volatile components 285, the mechanical strength of the object may be quite fragile, and handling of the object in this state should be performed with care. In an embodiment, each phase of the curing step 280 is performed in the same oven or kiln. In an embodiment, a handling tray is provided upon which the object can be processed to minimize handling damage.

FIG. 7 depicts a schematic representation of the formed object upon completion of the last step of the curing step 280, bond formation 330. Pore space 120 is created where the binder 230 and the pore former 240 were removed, and the fibers 210 are fused and bonded into the three dimensional matrix 110. The characteristics of the volatile components 285, including the size of the pore former 240 and/or the distribution of particle sizes of the pore former 240 and/or the relative quantity of the binder 230, together cooperate to predetermine the resulting pore size, pore size distribution, and pore interconnectivity of the resulting tissue scaffold 100. The bonding agent 220 and the bonds that form at overlapping nodes 610 and adjacent nodes 620 of the three dimensional matrix 110 provide for structural integrity of the resulting three-dimensional matrix 110.

Referring back to FIG. 4, the bond formation step 330 converts the nonvolatile components 275, including the bulk fiber 210, into the rigid three-dimensional matrix 110 of the tissue scaffold 100 while maintaining the pore space 120 created by the removal of the volatile components 275. The bond formation step 330 heats the non-volatile components 275 in an environment upon which the bulk fibers 210 bond to adjacent and overlapping fibers 210, and for a duration sufficient to form the bonds, without melting or altering the characteristics of the fibers 210, and thereby destroying the relative positioning of the non-volatile components 275. The bond formation environment and duration depends on the chemical composition of the non-volatile components 275, including the bulk fiber 210. For example, if titanium or titanium alloy-based fibers are used as the bulk fiber 210, the bond formation step 330 can be performed in a vacuum furnace at 10⁻³ torr and at a temperature of about 1,200°C. If alumina fibers are used as the bulk fiber 210, the bond formation step 330 can be performed in a static or air-purged kiln at atmospheric pressure and at a temperature of about 1,200°C to about 1,600°C. Other materials that may be used as the bulk fiber 210 can be heated to a temperature upon which solid state mass transfer occurs at the intersecting and overlapping nodes of the fiber structure, or liquid state bonding occurs, depending upon the composition of the non-volatile materials, in an environment that is conducive to the formation of such bonds, including but not limited to environments such as air, nitrogen, argon or other inert gas, and vacuum. If the fiber 210 is a bioactive glass fiber, the fiber may exhibit softening and a propensity for plastic deformation without fracture at a glass transition temperature. Glass materials typically have a devitrification temperature upon which the amorphous glass structure crystallizes. In an embodiment of the invention where bioactive glass is used to form the matrix 110, the bond formation temperature in the bond formation step 330 is in the working range between the glass transition temperature and the devitrification temperature of the bioglass materials. For example the bond formation temperature for 13-93 bioactive glass composition can be above the glass transition temperature of about 606°C and less than the devitrification temperature of about 851°C.

In the bond formation step 330, the formed object is heated to the bond formation temperature resulting in the formation of bonds at overlapping nodes 610 and adjacent nodes 620 of the fiber structure. If a bonding agent 220 is used, the bonds are formed at overlapping nodes 610 and adjacent nodes 620 of the fiber structure through a reaction of the bonding agent 220 in close proximity to the fibers 210, reacting with the fibers 210 to form bonds. In the bond formation step 330, the material of the fibers 210 may participate in a chemical reaction with the bonding agent 220, or the fibers 210 may remain inert with respect to a reaction of the bonding agent 220. Further still, the bulk fibers 210 may be a mixture of fiber compositions, with a portion, or all of the fibers 210 participating in a reaction forming bonds to create the three-dimensional matrix 110. Because the formed object is fabricated using a first batch 130 and a second batch 135 with each batch having properties that yield distinct properties, the bond formation step 330 must be configured to form bonds in the respective regions of the the formed object associated with the first batch 130 and the second batch 135. Additionally, due to the forming step 140, there may be mixing and blending of the respective batch materials to form a region that yields properties that range between the region associated with the first batch 130 and the region associated with the second batch 135, and thus, the bond formation step 330 must be configured to also form bonds in this transition region.

The duration of the bond formation step 330 depends on the temperature profile during the bond formation step 330, in that the time at the bond formation temperature of the fibers 210 is limited to a the duration so that the relative position of the non-volatile components 275, including the bulk fibers 210, does not significantly change. The pore size, pore size distribution, and interconnectivity between the pores in the formed object are determined by the relative position of the bulk fibers 210 by the volatile components 285. While the volatile components 285 are likely burned out of the formed object by the time the bond formation temperature is attained, the relative positioning of the fibers 210 and non-volatile components 275 are not significantly altered. The formed object will likely undergo slight or minor densification during the bond formation step 330, but the control of pore size and distribution of pore sizes can be maintained, and therefore predetermined by selecting a particle size for the pore former 240 that is slightly oversize or adjusting the relative quantity of the volatile components 285 to account for the expected densification.

The bonds formed between overlapping and adjacent nodes of the intertangled fibers forming the three-dimensional matrix 110 can be sintered bonds having a composition substantially the same as the composition of the bulk fibers 210. The bonds can also be the result of a reaction between the bulk fibers 210 and the bonding agent 220 to form a bonding phase having a composition that is substantially the same, or different than the composition of the bulk fiber 210. Due to the regulatory requirements relating to the approval of materials for use as a medical device or implant, it may be desirable to use approved material compositions as raw materials that are not significantly altered by the device fabrication methods and processes. Alternatively, it may be desirable to use raw materials that are precursors to an approved material composition, that form the desired composition during the device fabrication methods and processes. The present invention provides a tissue scaffold device that can be either fabricated using a variety of medically approved materials, or fabricated into a medically-approved material composition.

The tissue scaffold 100 of the present invention exhibits controlled pore interconnectivity because of the ability to control the pore morphology by specifying characteristics of the non-volatile components 275 and volatile components 285. For example, the fiber length distribution can exhibit a mode that is greater than the pore former diameter to enhance pore interconnectivity in that the fibers exhibiting this mode will extend from one pore to another, with the space between adjacent fibers creating pore interconnectivity. Further, the fiber diameter being less than the pore former particle size can ensure closer packing of pore former particles to provide improved pore interconnectivity.

The properties of the tissue scaffold 100 can be controlled and adjusted or optimized for a specific application through the manipulation of various parameters in the regions associated with the respective first batch 130 and the second batch 135 and/or through the manipulation of various parameters and characteristics of the raw materials including the non-volatile components 275 and the volatile components 285 in each of the first batch 130 and the second batch 135. For example, in a load bearing application, the elastic modulus of the tissue scaffold 100 can be optimized and controlled in various ways as described herein.

A tissue scaffold in a load bearing application preferably distributes load evenly over a large area so that stress is continuously transmitted to the surrounding tissue in order to encourage healthy bone formation throughout the interface. The property of the tissue scaffold that primarily influences the effectiveness of the scaffold in transmitting continuous stress is elastic modulus. When the elastic modulus of the tissue scaffold is closely matched to the elastic modulus of the surrounding tissue, the stress transmitted through the scaffold to the surrounding tissue stimulates the growth of healthy new tissue. If the elastic modulus of the scaffold is relatively greater than the elastic modulus of the surrounding tissue, regenerated tissue that grows into the scaffold is effectively shielded from stress resulting in a disturbing phenomenon known as bone resorption according to Wolff's Law (bone adapting itself to stress reduction by reducing its mass, either by becoming more porous or by getting thinner). If the elastic modulus of the scaffold is excessively less than the elastic modulus of the surrounding tissue stress cannot be effectively transmitted to the surrounding tissue without deformation of the scaffold and exerting excessive stress and strain on newly formed tissue.

The method and apparatus of the present invention permits the fabrication of an ideally matched elastic modulus through the control of various factors for a given material composition in a region associated with the first batch 130 and/or the second batch 135. Generally, variation of fiber 210 characteristics, variation of the characteristics of the volatile components 285, variation of the bonding agent 220 characteristics in the respective batches, and control of the environment of the curing step 280 can result in optimization of the resulting strength, porosity and elastic modulus of the scaffold 100.

Fiber characteristics include composition, diameter, length directly impact the strength and flexibility of the scaffold. Compositional influences arise from the inherent physical characteristics of the fiber materials, such as tensile strength and elastic modulus, including factors such as grain boundaries and brittleness of the material. The diameter of the fiber can impact the resulting strength and flexibility of the scaffold in that thicker fibers tend to be stronger and more stiff. Longer fibers can provide increased flexibility. Additionally, the diameter and length of the fiber, individually or collectively, directly influence the natural packing density of the fiber materials. The greater the natural packing density of the fiber, the more fiber-to-fiber connections are possible in the resulting scaffold. When fiber-to-fiber connections are increased, the strength and modulus of the scaffold is generally increased.

The bonding agent 220, when used, can influence the resulting strength and flexibility of the scaffold. The bonding agent 220 can increase the number of fiber-to-fiber connections in the matrix which will increase the resulting strength and change the elastic modulus accordingly. Additionally, the relative quantity of the bonding agent 220 will increase the amount of non-volatile components relative to the volatile components, which will impact the porosity. Generally, high porosity, with all else being the same, will result in reduced strength. The composition of the bonding agent 220 will impact the strength and flexibility of the resulting scaffold in that the inherent physical characteristics, such as tensile and compressive strength and elastic modulus, are imputed to the resulting scaffold. The particle size of the bonding agent 220 can influence the strength and modulus in that larger particles have a tendency to reside at the intersections of fibers, resulting in more material available to bridge adjacent fibers and joint them into the bonded matrix. Smaller particles have a tendency to remain in the same relative position when the binder is burned out so that it adheres to the surface of the fiber to alter the chemical and physical properties of the fiber. Additionally, the smaller particles and/or smaller relative quantities of the bonding agent 220 may result in fewer fiber-to-fiber bonds, which will reduce the strength and reduce the elastic modulus of the resulting scaffold.

Volatile component characteristics can influence the resulting strength and flexibility of the scaffold. Pore formers can control the size and distribution of the interconnecting pores throughout the scaffold, as described in more detail above. With respect to the influence on mechanical properties of the scaffold 100, an increase in the amount of volatile components, including increased relative quantities of pore former, can impact the strength and lower the elastic modulus of the scaffold, with all else remaining the same. Furthermore, there are secondary interactions with the variables associated with fiber diameter and fiber length with regard to the natural packing density of the fiber material. The volatile components, when mixed with the non-volatile components, can increase bundling of the fibers in that two or more fiber lengths will align substantially adjacent to additional fibers, and bond together along the fiber length, effectively increasing the cross-sectional area of the "struts" that form the matrix of the scaffold. Regions of bundled fiber in this manner will effectively impact the strength and elastic modulus of the scaffold 100.

Processing parameters selected during the method of forming the scaffold 100 using the first batch 130 and the second batch 135 can influence the properties of the scaffold. For example, the curing step 280 environment parameters include heating rate, heating temperature, curing time, and heating environment, such as vacuum, inert gas (nitrogen, argon, etc.), forming gas (reducing environment) or air. Each or combinations of each can influence the number and relative strength of fiber-to-fiber bonds throughout the scaffold.

Additional factors for controlling and optimizing the porosity/ strength relationship and the elastic modulus of the scaffold 100 include specific characteristics of the raw materials combined with the certain fabrication process steps that can influence a general alignment of the fibers. Fiber alignment in at least one region can induce texture as a property that can induce directionally preferential tissue growth within the scaffold. The mixing step 260 and the forming step 140 can be adapted to provide a formed object that aligns the fibers substantially in one direction for any one or both of the regions associated with the first batch 130 and the second batch 135. For example, the use of an extrusion process in the forming step 140 can impart a general alignment of the fibers of the mixture in the direction of extrusion. The physical characteristics of the resulting scaffold 100 can exhibit an elastic modulus that is a function of the orientation of the device, in that the compressive strength and elastic modulus can be relatively high in the extrusion direction, while lower in the direction perpendicular to the extrusion direction. A spinal implant that is used to fuse vertebrae can be designed with these variable characteristics to optimize the load bearing and weight sharing features of the scaffold to ensure the growth of healthy tissue. Fiber orientation may be desirable in certain applications where vascularization into the scaffold is necessary. The oriented fibers will induce pore morphology that exhibits a preferential direction parallel to the fibers. In an application where the scaffold 100 is to fuse bone tissue, the vascularization link between the adjoining bones can be effectively bridged by the scaffold of the present invention.

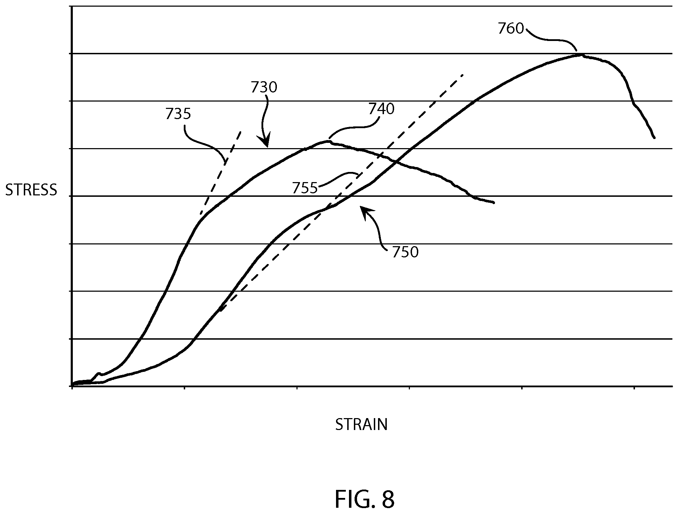

FIG. 8 depicts the stress-strain curves 720 resulting from a compression test of two exemplary batches of material formed into scaffolds according to the present invention that demonstrates the effect of change in strength and elastic modulus of a scaffold through the addition of a bonding agent during fabrication. Both samples were fabricated from individual batches in the methods described herein above using titanium Ti6Al4V alloy fiber having an average diameter of approximately 63 µm. The first sample was fabricated from a batch prepared by mixing 3 grams of fiber cut to 0.045" length with 1 gram of fiber cut to 0.010" length with 0.25 grams HPMC as an organic binder and 1 gram of PMMA with a particle size of about 100 µm as a pore former and approximately 1.5 grams of deionized water, adjusted as necessary to provide a plastically formable mixture. The mixture was extruded into a 10 mm diameter rod and dried in a convention oven. The volatile components were burned out and then the scaffold was heat treated at 1,400°C at 10⁻³ torr vacuum for two hours to create a scaffold having a porosity of 70%. The second sample was fabricated from a similar batch prepared with the only change being an addition of 0.25 grams titanium powder with a particle size of less than 325 µm as a bonding agent 220, with the resulting porosity of 67%. Referring to FIG. 7, the stress-strain curve for the first sample 730 (no bonding agent) exhibits a first elastic modulus 735 and a first peak strength value 740. The second sample 750 (with bonding agent) exhibits a second elastic modulus 755 that is approximately 65% less than the first elastic modulus 735 and a second peak strength value 760 that is approximately 34% greater than the first strength value 740. In an embodiment of the invention, a tissue scaffold 100 is formed using the first batch 130 as the batch from the first sample associated with a first region of the tissue scaffold and using the second batch 135 as the batch from the second sample associated with a second region of the tissue scaffold. The object is formed by co-extrusion of the two respective batches. The first region will exhibit the first elastic modulus 735 and the second region will exhibit the second elastic modulus 755. A third region (not shown) will exhibit an elastic modulus that is a gradient value between the first elastic modulus 735 and the second elastic modulus 755.

Furthermore, variations of any one or variations in any combination of the above parameters can be made to attain an optimized or desired strength and elastic modulus, porosity, and pore size distribution in a first batch 130 and an optimized or desired strength and elastic modulus, porosity, and pore size distribution in a second batch 135 that can be used to form an object that is cured into a tissue scaffold for an intended application. Furthermore, the strength, elastic modulus, porosity and pore size distribution, and other mechanical and physical properties can be adjusted in any one of the first batch 130 or the second batch 135 for other applications, non-limiting examples of which are herein described.

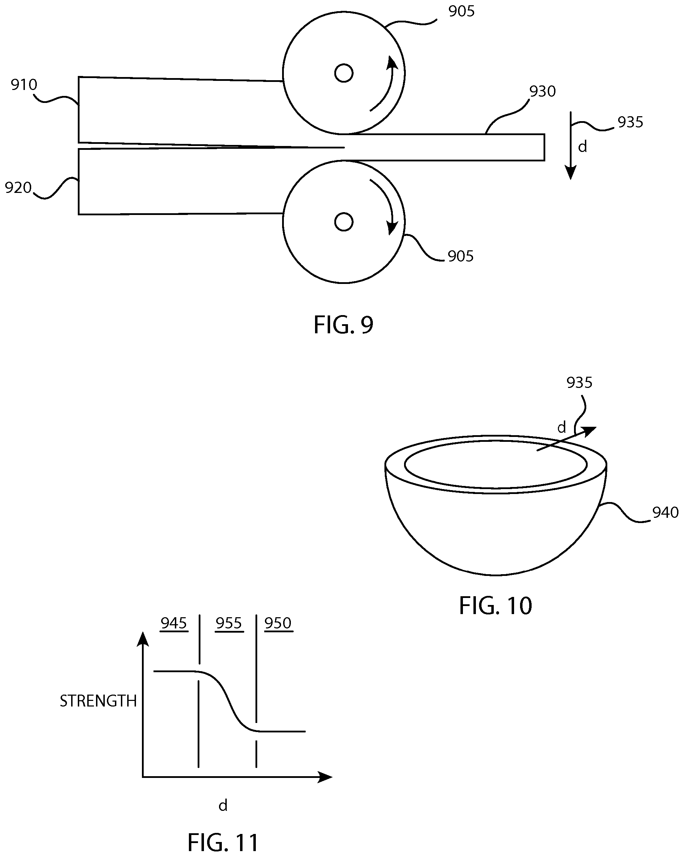

Referring to FIG. 9 an embodiment of the forming step 140 is shown. In this embodiment, the first batch 130 is prepared as a first flat sheet 910 from raw materials that yield a low porosity - high compressive strength material, such as through short lengths of small diameter fiber material 210 and/or with high relative amounts of a bonding agent 220 and/ or low relative amounts of pore former 240. A second batch 135 is prepared as a second flat sheet 920 from raw materials that yield a high porosity - low compressive strength material, such as though longer lengths of thicker diameter fiber 210 and/or lower relative amount of bonding agent 220 and/or higher relative amounts of pore former 240. The first flat sheet 910 and the second flat sheet 920 are passed through a pair of rollers 905 that reduce the section thickness of the first flat sheet 910 and second flat sheet 920 to produce a third flat sheet 930. The pressure and displacement of the first flat sheet 905 and the second flat sheet 920 forces at least a portion of the respective materials together in a transitional region resulting third flat sheet 930 will exhibit a gradient composition 935 over the thickness "d" that will result in a gradient of properties once cured at step 145.

FIG. 10 depicts an acetabular cup 940 that can be formed from compression molding of the third flat sheet 930 of FIG. 9 with the gradient in composition 935 oriented as shown in FIG. 10. When the third flat sheet 930 is formed into the acetabular cup 940 and cured at step 145, the gradient in composition 935 of the respective first batch 130 and the second batch 135 will result in properties of the device exhibiting a gradient in properties, as represented by FIG. 11. FIG. 11 depicts the gradient in properties, shown as a gradient in compressive strength, starting from a region of high strength 945 on the interior of the acetabular cup 940 to a region of low strength 950 (at high porosity) on the exterior of the acetabular cup 940 with a region that is a gradient of compressive strength 955 in a transitional region between the interior region and the exterior region.

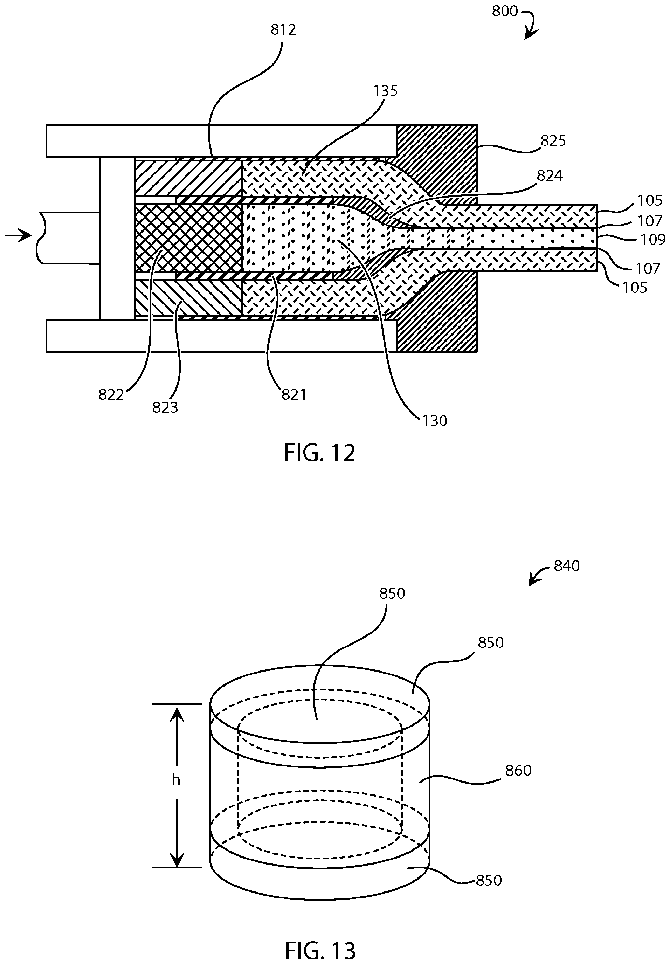

Referring now to FIG. 12, a cross section of an extrusion system 800 adapted to perform the forming step 140 as co-extrusion of a first batch 130 and a second batch 135 is shown according to the present invention. In this illustrative example, a first batch 130 is prepared and inserted into the central chamber 821. A second batch 135 is prepared and placed in the outer chamber 812 that is an annular cylinder surrounding the central chamber 821. An external extrusion die 825 determines the shape of the extruded article, and an internal extrusion die 824, supported by the external die 825 by support members (not shown for clarity). An outer piston 823 is placed in contact with the second batch 135 and a central piston 822 is placed in contact with the first batch 130, and the application of hydraulic pressure simultaneously on the outer piston 823 and the central piston 822 will force both the first batch 130 through the internal die 824 and the second batch 135 will flow over the first batch 130 and through the external die 825 forming an extruded article with a first region 105 and a second region 109 with a third region 107 being formed during the extrusion process as the two batch materials are joined by the extrusion pressure. The extruded object can then be cured according to step 145 to exhibit a gradient in properties commensurate with the raw materials used to prepare the first batch 130 and the second batch 135.

One skilled in the art will appreciate that alternative methods of co-extrusion can be performed using other systems, including pre-loading an extrusion barrel with a slug prepared as a coaxial arrangement of first batch 130 and a second batch 135, or extruding a first article with a first batch, and extruding the second batch 135 over the first batch.

Referring to FIG. 13, another embodiment of the forming step 140 is shown. In this embodiment, the first batch 130 is prepared and the second batch 135 is prepared and a rapid prototype dispenser is used to fabricate a tissue scaffold 840 using the first batch 130 and the second batch 135. In this embodiment, the first batch 130 is dispensed to a first region 850 at the top, bottom, and central core of the scaffold 840. The second batch 135 is dispensed to a second region 860 with the dispensing pressure of the second batch 135 inducing flow and joining of the two materials at the interface. The scaffold 840 is then cured as described above with reference to step 145 to provide a gradient in properties between the first region 850 and the second region 860. A tissue scaffold according to the embodiment shown at FIG. 13 can be used as a spinal implant or intervertebral (IVB) device that can be implanted in the spine to maintain the intervertebral space corresponding to the height (h) of the implant. Complex shapes with a wide range of regions can be formed using the method of forming 140 depicted in this embodiment of the invention.

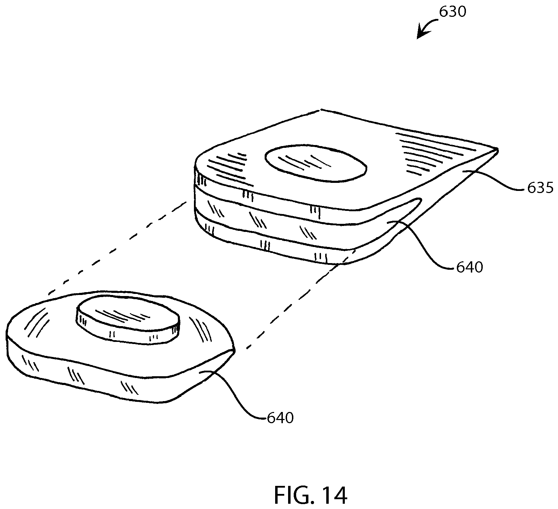

FIG. 14 depicts an osteotomy implant 630 that may be generally described as a wedge designed to conform to an anatomical cross section of, for example, a tibia, thereby providing mechanical support to a substantial portion of a tibial surface. The osteotomy wedge 630 is formed from a first batch 130 prepared as a first region 640 that may, for example, be fabricated in a low porosity, high compressive strength-yielding mixture, surrounded by a second batch 135 provided as a second region 635 that may, for example, be fabricated in a high porosity-yielding mixture. The osteotomy implant 630 can be fabricated by compression or injection molding the first region 640 and compression or injection molding the second region 635 with the first region 640 being first placed into position in the mold so that the second batch 135 is directed to plastically flow around and join to the first region 640. Alternatively, the respective regions can be each formed using investment casting techniques. When cured as described above with reference to step 145, the resulting osteotomy implant 630 can exhibit a central core having a high compressive strength with a low compressive strength (but highly porous and osteoconductive) region that permits in-growth of tissue to incorporate the implant 630 into the tibia.

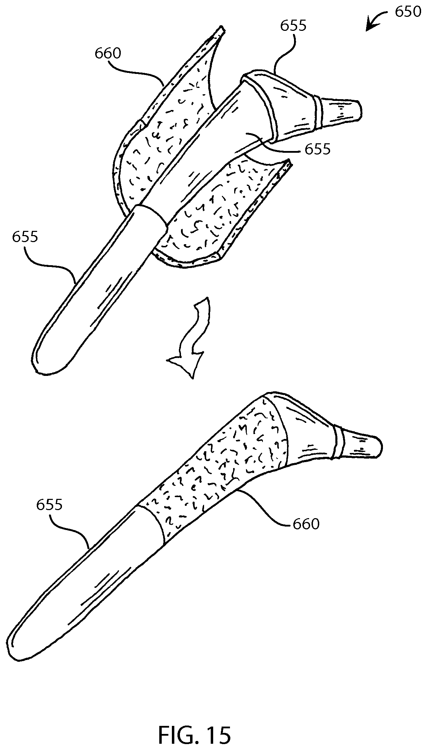

FIG. 15 depicts an exploded view of a femoral hip stem 650 having a first region 655 and a second region 660. In an illustrative embodiment of the present invention, the hip stem 650 can be fabricated from a first batch 130 that is prepared from fiber materials yielding a low porosity, low elastic modulus material that closely matches the elastic modulus of natural bone. The second region 660 can be fabricated from a second batch 135 that is prepared from fiber materials yielding a high porosity, low elastic modulus material that yields a highly osteoconductive surface into which natural bone can grow. The first batch 130 can be cast to shape using compression molding techniques, with the second batch 135 can be cast to the shaped article by a successive compression molding technique. When the hip stem 650 is then cured according to the above description with regard to step 145, the hip stem 650 will exhibit highly porous region 660 for bone in-growth surrounding a highly flexible central core region 655, with a third region resulting from the mixture of the first batch material 130 and the second batch material 135 at an interfacial region during the successive compression molding operation. The fabrication method for this illustrative embodiment can be successive compression molding operations to form the first region 655 followed by a molding step of the entire implant with the second region 660 applied to the device. Alternatively, the hip stem 650 can be fabricated using hot isostatic pressure forming methods.

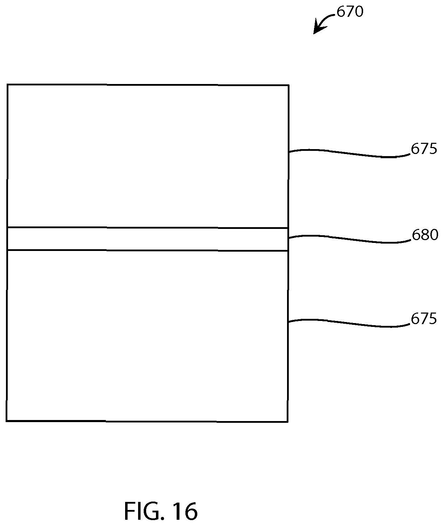

FIG. 16 depicts a cross section of a tissue scaffold 670 having a gradient in properties according to the present invention where at least on of the two regions provides a barrier to osteoconductivity. In this embodiment the scaffold 670 has a first region 675 fabricated from a first batch 130 prepared from fiber materials yielding a high porosity material and a second region 680 fabricated from a second batch 135 prepared from fiber materials yielding a low porosity material. In this embodiment, the highly porous first region 675 is separated by the low porosity second region 680 whereby tissue in-growth that may occur in the distal end of the implant 670 will be limited and inhibited at the second region 680. An indication for which the tissue scaffold 670 of this embodiment can be used is where cartilage tissue in-growth is desired but such in-growth must be limited to facilitate in-growth of bone tissue on an opposing distal end of the implant 670.

### Examples

The following examples are provided to further illustrate and to facilitate the understanding of the disclosure. These specific examples are intended to be illustrative of the disclosure and are not intended to be limiting in any way.

In a first exemplary embodiment a scaffold is formed from titanium fiber. A first batch is prepared by mixing 4 grams of Ti6Al4V alloy fiber having an average diameter of approximately 0.0025" chopped into lengths of approximately 0.045" with 0.25 grams HPMC as an organic binder and no pore former, with water sufficient to form an extrudable batch. A second batch is prepared by mixing 2 grams of Ti6Al4V alloy fiber having an average diameter of approximately 0.0025" chopped into lengths of approximately 0.045" with 2 grams of Ti6Al4V alloy fiber having an average diameter of approximately 0.0025" chopped into lengths of approximately 0.100" with 0.25 grams HPMC as an organic binder and 0.5 grams PMMA particles having a particle size of approximately 600 µm as a pore former, with water sufficient to form an extrudable batch. The first batch and the second batch are loaded into a co-extrusion system as described above with reference to FIG. 12 extruding a 12 mm diameter rod with a 6 mm core, the first batch associated with the core and the second batch forming the outer region of the scaffold. The scaffold was dried and the volatile components were burned out and then heat treated at 1,400°C at 10-3 torr vacuum for two hours. The porosity was determined to be 64% in the core region and 70% in the outer region, with a transitional region exhibiting a gradient between the two values of porosity.

In a second exemplary embodiment a scaffold is formed from titanium fiber. A first batch is prepared by mixing 4 grams of Ti6Al4V alloy fiber having an average diameter of approximately 0.0025" chopped into lengths of approximately 0.045" with 0.25 grams HPMC as an organic binder and no pore former, with water sufficient to form an extrudable batch. A second batch is prepared by mixing 4 grams of Ti6Al4V alloy fiber having an average diameter of approximately 0.0025" chopped into lengths of approximately 0.045" with 1 gram titanium powder having a particle size of 325 µm or less as a bonding agent with 0.25 grams HPMC as an organic binder and 1 gram PMMA particles having a particle size of approximately 300 µm as a pore former, with water sufficient to form an extrudable batch. The first batch and the second batch are loaded into a co-extrusion system as described above with reference to FIG. 12 extruding a 12 mm diameter rod with a 6 mm core, the first batch associated with the core and the second batch forming the outer region of the scaffold. The scaffold was dried and the volatile components were burned out and then heat treated at 1,400°C at 10-3 torr vacuum for two hours. The elastic modulus was determined to be 0.54 GPa in the core region and 1.02 GPa in the outer region, with a transitional region exhibiting a gradient between the two values of porosity.

In a third exemplary embodiment a scaffold is formed from titanium fiber. A first batch is prepared by mixing 3 grams of Ti6Al4V alloy fiber having an average diameter of approximately 0.0025" chopped into lengths of approximately 0.045" and 1 gram of Ti6Al4V alloy fiber having an average diameter of 0.0025" chopped into lengths of approximately 0.100" with 0.25 grams HPMC as an organic binder and no pore former, with water sufficient to form an extrudable batch. A second batch is prepared by mixing 3 grams of Ti6Al4V alloy fiber having an average diameter of approximately 0.0025" chopped into lengths of approximately 0.045" and 1 gram of Ti6Al4V alloy fiber having an average diameter of 0.0025" chopped into lengths of approximately 0.100" with 0.25 grams titanium powder having a particle size of 325 µm or less as a bonding agent with 0.25 grams HPMC as an organic binder and 1 gram PMMA particles having a particle size of approximately 300 µm as a pore former, with water sufficient to form an extrudable batch. The first batch and the second batch are loaded into a co-extrusion system as described above with reference to FIG. 12 extruding a 12 mm diameter rod with a 6 mm core, the first batch associated with the core and the second batch forming the outer region of the scaffold. The scaffold was dried and the volatile components were burned out and then heat treated at 1,400°C at 10-3 torr vacuum for two hours. The compressive strength was determined to be 1.10 MPa in the core region and 66 MPa in the outer region, with a transitional region exhibiting a gradient between the two values of porosity.

## Claims

1. An intervertebral device comprising:
a tissue engineering scaffold having a height sized for engagement within a space between adjacent vertebrae and to maintain the intervertebral space corresponding to the height of the tissue engineering scaffold, the tissue engineering scaffold having a top surface, a bottom surface, and a central core;
the tissue engineering scaffold having a first region (850) of interconnected and bonded fiber comprising the top surface, the bottom surface, and the central core;
the tissue engineering scaffold having a second region (860) of interconnected and bonded fiber comprising an annulus around the central core; wherein the first region (850) has a property exhibiting a first value; the second region (860) has a property exhibiting a second value; and the first region (850) and the second region (860) are joined to form a continuous structure; and
a third region of interconnected and bonded fiber between the first region (850) and the second region (860), the third region having the property exhibiting a gradient value between the first value and the second value.

2. The intervertebral device according to claim 1 wherein the property is porosity.

3. The intervertebral device according to claim 1 wherein the property is elastic modulus.

4. The intervertebral device according to claim 1 wherein the property is strength.

5. The intervertebral device according to claim 1 wherein the property is composition.

6. The intervertebral device according to claim 1 wherein the property is bioactivity.

7. The intervertebral device according to claim 1 wherein one of the first and second regions provides osteoconductivity.

## Patentansprüche

1. Eine Zwischenwirbel-Vorrichtung, aufweisend:
ein Gewebezüchtungsgerüst, das eine Höhe hat, die für ein Eingreifen innerhalb eines Raumes zwischen benachbarten Wirbeln bemessen ist, und zum Halten des Zwischenwirbelraumes entsprechend der Höhe des Gewebezüchtungsgerüstes, wobei das Gewebezüchtungsgerüst eine obere Fläche, eine untere Fläche und einen zentralen Kern hat,
wobei das Gewebezüchtungsgerüst einen ersten Bereich (850) einer vernetzten/ineinandergreifenden und verbundenen Faser hat, der die obere Fläche, die untere Fläche und den zentralen Kern aufweist,
das Gewebezüchtungsgerüst einen zweiten Bereich (860) einer vernetzten/ineinandergreifenden und verbundenen Faser hat, der einen Kranz um den zentralen Kern aufweist,
wobei der erste Bereich (850) eine Eigenschaft hat, die einen ersten Wert aufweist, der zweite Bereich (860) eine Eigenschaft hat, die einen zweiten Wert aufweist, und der erste Bereich (850) und der zweite Bereich (860) verbunden sind, um eine durchgehende Struktur auszubilden, und
einen dritten Bereich einer ineinandergreifenden/vernetzten und verbundenen Faser zwischen dem ersten Bereich (850) und dem zweiten Bereich (860), wobei der dritte Bereich die Eigenschaft hat, die einen Gradientenwert zwischen dem ersten Wert und dem zweiten Wert aufweist.

2. Die Zwischenwirbel-Vorrichtung gemäß Anspruch 1, wobei die Eigenschaft die Porosität ist.

3. Die Zwischenwirbel-Vorrichtung gemäß Anspruch 1, wobei die Eigenschaft der Elastizitätsmodul ist.

4. Die Zwischenwirbel-Vorrichtung gemäß Anspruch 1, wobei die Eigenschaft die Festigkeit ist.

5. Die Zwischenwirbel-Vorrichtung gemäß Anspruch 1, wobei die Eigenschaft die Zusammensetzung ist.

6. Die Zwischenwirbel-Vorrichtung gemäß Anspruch 1, wobei die Eigenschaft die Bioaktivität ist.

7. Die Zwischenwirbel-Vorrichtung gemäß Anspruch 1, wobei einer von dem ersten und dem zweiten Bereich Osteokonduktivität bereitstellt.

## Revendications

1. Dispositif intervertébral comprenant :
un support d'ingénierie tissulaire ayant une hauteur dimensionnée pour la mise en prise à l'intérieur d'un espace entre des vertèbres adjacentes et pour maintenir l'espace intervertébral correspondant à la hauteur du support d'ingénierie tissulaire, le support d'ingénierie tissulaire ayant une surface supérieure, une surface inférieure et une âme centrale ;
le support d'ingénierie tissulaire ayant une première région (850) de fibres interconnectées et reliées comprenant la surface supérieure, la surface inférieure et l'âme centrale ;
le support d'ingénierie tissulaire ayant une deuxième région (860) de fibres interconnectées et reliées comprenant un espace annulaire autour de l'âme centrale ;
dans lequel la première région (850) a une propriété laissant apparaître une première valeur ; la deuxième région (860) a une propriété laissant apparaître une seconde valeur ; et la première région (850) et la deuxième région (860) sont assemblées pour former une structure continue ; et
une troisième région de fibres interconnectées et reliées entre la première région (850) et la deuxième région (860), la troisième région ayant la propriété laissant apparaître une valeur de gradient entre la première valeur et la seconde valeur.

2. Dispositif intervertébral selon la revendication 1, dans lequel la propriété est la porosité.

3. Dispositif intervertébral selon la revendication 1, dans lequel la propriété est le module d'élasticité.

4. Dispositif intervertébral selon la revendication 1, dans lequel la propriété est la résistance.

5. Dispositif intervertébral selon la revendication 1, dans lequel la propriété est la composition.

6. Dispositif intervertébral selon la revendication 1, dans lequel la propriété est la bio activité.

7. Dispositif intervertébral selon la revendication 1, dans lequel l'une parmi la première et la deuxième région fournit l'ostéoconductivité.
